# EUROPEAN PATENT APPLICATION

(11) **EP 1 331 006 A1**
(43) Date of publication of application: **30.07.2003**
(21) Application number: 03001728.9
(22) Date of filing: 27.01.2003
(51) Int. Cl.: A61K 35/70, A61K 35/72, A61K 35/74, A23K 1/00, A23K 1/16, A23L 1/30, A23L 1/28, A23L 2/00, C12N 1/16, C12N 1/20

(54) **Dietary supplements comprising growth media**

(30) Priority: 25.01.2002 US 57649
(71) Applicant: PHYTOCHEM HOLDING AS, 3209 Sandefjord (NO)
(72) Inventor: Paaske Sverre, N-6422 Molde (NO); Endresen Curt, N-6422 Molde (NO)
(74) Representative: Glawe, Delfs, Moll & Partner

(57) **Abstract**

The present invention relates to dietary supplements. In particular, the present invention provides compositions and methods utilizing dietary supplements comprising fungus and bacteria co-culture growth media. In preferred embodiments, the bacteria is *Gluconacetobacter europaeus* or *Bacillus pumilus* or a combination thereof and the fungus is *Zygosaccharomyces*. The microorganisms are preferably substantially removed from the growth media. In preferred embodiments, the growth media is dried down, lyophilized, or spray dried to provide a powder.

## Description

### FIELD OF THE INVENTION

The present invention relates to dietary supplements. In particular, the present invention provides compositions and methods utilizing dietary supplements derived from fungi and bacteria co-culture growth media.

### BACKGROUND OF THE INVENTION

Nutrition is a critical determinant of immunological competence and of the individual's ability to resist infection and physiological stresses. The health of individuals is affected by poor decisions in society's management of new technology over the past five decades. In particular, the routine use of antibiotics, the consumption of processed foods and the pollution of the environment have resulted in multiple adverse influences upon health. Among these may be included the proliferation of new strains of bacteria and viruses that are resistant to existing antibiotic and antiviral agents, compromised immune systems resulting from chemical pollutants in food, water and air, as well as impaired ability to repair tissue and muscle. Additionally, emotional and physical stresses from employment, family, exercise and the natural effects of aging reduce the effectiveness of the immune system and tissue repair processes.

The continuing widespread use of antibiotics in medicine and agriculture reinforces the selective pressures that increase the types and extent of antibiotic-resistant microbes in the environment, which then substantially increases the cost of treating infection. Moreover, administration of antibiotics frequently causes disruption of the normal bacterial flora colonizing the individual's digestive tract, with results that are particularly undesirable in weakened patients.

Increased attention is being given to nutrition and its critical role in health and in fighting diseases and other infections. A variety of nutritional compositions have been administered in the past to subjects to stimulate the immune system and minimize the risk of infections. Nutritional adjunctive therapy given to patients either enterally or parenterally has been shown to be efficacious in reversing catabolism and stimulating anabolism. This improvement in the metabolic state of the patient is believed to be critical to the healing process and resistance to further infections.

The physiological rigors to which an individual's body is exposed in the modern environment, which include the chemical pollutants and antibiotic-resistant microbes discussed above, indicate the advisability of enhancing the immune system to facilitate the body's abilities to resist and cope with infection, and to assist the natural, self-healing processes. Two groups of individuals are particularly susceptible to infection and the side effects of treatment: young children and the aged. These individuals may respond poorly to physiological or environmental challenges because they typically possess immune systems that are, in young children and in the aged respectively, immature or damaged. Consequently, natural stimulation of these individuals' immune systems is particularly desirable.

Stimulation of the immune system may occur if the appropriate proteins are absorbed into an individual's bloodstream. Yet these proteins are not only degraded rapidly by the acidic and enzymatic conditions of the stomach and intestine but they are also expensive to obtain, even in the quantities and formats used for experimental demonstrations. Thus attempts to formulate an effective dietary supplement able to generate and maintain a state of immune stimulation in an individual have been unsuccessful.

Accordingly, what is needed in the art are dietary supplements that in addition to nutritional characteristics, also aid the body's capacity to generally stimulate the immune system, resist fresh infection and to suppress existing infection or to increase tissue repair and healing.

### SUMMARY OF THE INVENTION

The present invention relates to dietary supplements. In particular, the present invention provides compositions and methods utilizing dietary supplements derived from fungi and bacteria co-culture growth media.

For example, in some embodiments, the present invention provides a dietary supplement comprising growth medium from at least one microorganism. In some embodiments, the microorganism is a fungi. In other embodiments, the fungi is present as a co-culture. In some preferred embodiments, the co-culture is a symbiotic co-culture. In some embodiments, the fungi is *Zygosaccharomyces.* In some preferred embodiments, the co-culture comprises at least one bacteria. In some embodiments, the bacteria is selected from a group including, but not limited to, *Gluconacetobacter europaeus* and *Bacillus pumilus.* In some embodiments, the growth medium is dry growth medium. In some embodiments, the supplement further comprises one or more additional components including, but not limited to, dry red wine extract, dry shizanda extract, alfalfa, and papain. For example, in some embodiments, the supplement comprises dry growth medium and dry red wine at a ratio of approximately 2.35:1. In other embodiments, the supplement comprises dry growth medium and dry shizandra extract at a ratio of approximately 4.7:1. In still further embodiments, the supplement comprises dry growth medium, alfalfa, and papain at a ratio of approximately 7.8:1.67:1. In yet other embodiments, the present invention provides a foodstuff comprising the dietary supplement.

The present invention additionally provides a food comprising growth medium from at least one microorganism. In some embodiments, the microorganism is a fungi. In some preferred embodiments, the fungi is present as a co-culture. In some embodiments, the co-culture is a symbiotic co-culture. In some embodiments, the fungi is *Zygosaccharomyces.* In some embodiments, the co-culture comprises at least one bacteria. In some embodiments, the food is heated. In some embodiments, the growth medium is added to the food before the food is heated. In some embodiments, the food is a bread. In other embodiments, the food is a beverage. In some embodiments, the food comprises 3-5 g of dry growth media per 1000 g of food. In some embodiments, the bacteria is selected from a group including, but not limited to, *Gluconacetobacter europaeus* and *Bacillus pumilus.*

In other embodiments, the present invention additionally provides an animal feed comprising growth medium from at least one microorganism. In some embodiments, the microorganism is a fungi. In some preferred embodiments, the fungi is present as a co-culture. In some embodiments, the co-culture is a symbiotic co-culture. In some embodiments, the fungi is *Zygosaccharomyces.* In some embodiments, the co-culture comprises at least one bacteria. In some embodiments, the bacteria is selected from a group including, but not limited to, *Gluconacetobacter europaeus* and *Bacillus pumilus.*

The present invention further provides a method of stimulating immune response in a cell, comprising providing a dietary supplement comprising growth medium from at least one microorganism; and a cell; and administering the growth media to the cell under conditions such that an immune response is stimulated in the cell. In some embodiments, the immune response comprises a T-cell immune response. In some embodiments, the cell is a T-lymphocyte. In some embodiments, the T-lymphocyte is part of an animal. In some embodiments, the animal is a human. In some embodiments, the micro organism is a fungi. In some preferred embodiments, the fungi is present as a co-culture. In some embodiments, the co-culture is a symbiotic co-culture. In some embodiments, the fungi is *Zygosaccharomyces.* In some embodiments, the co-culture comprises at least one bacteria. In some embodiments, the bacteria is selected from a group including, but not limited to, *Gluconacetobacter europaeus* and *Bacillus pumilus.*

### DESCRIPTION OF THE FIGURE

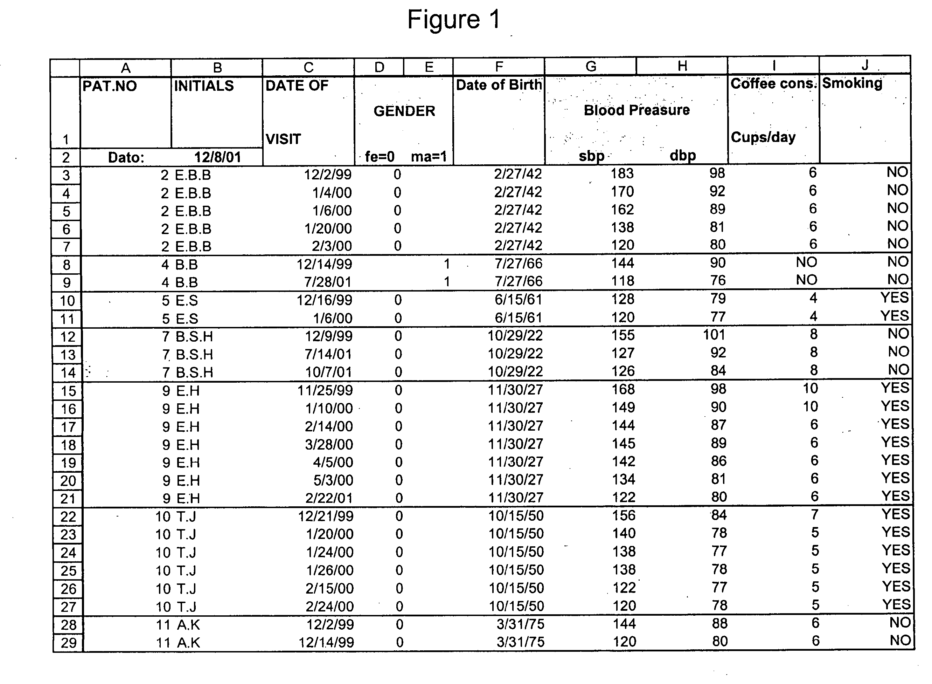

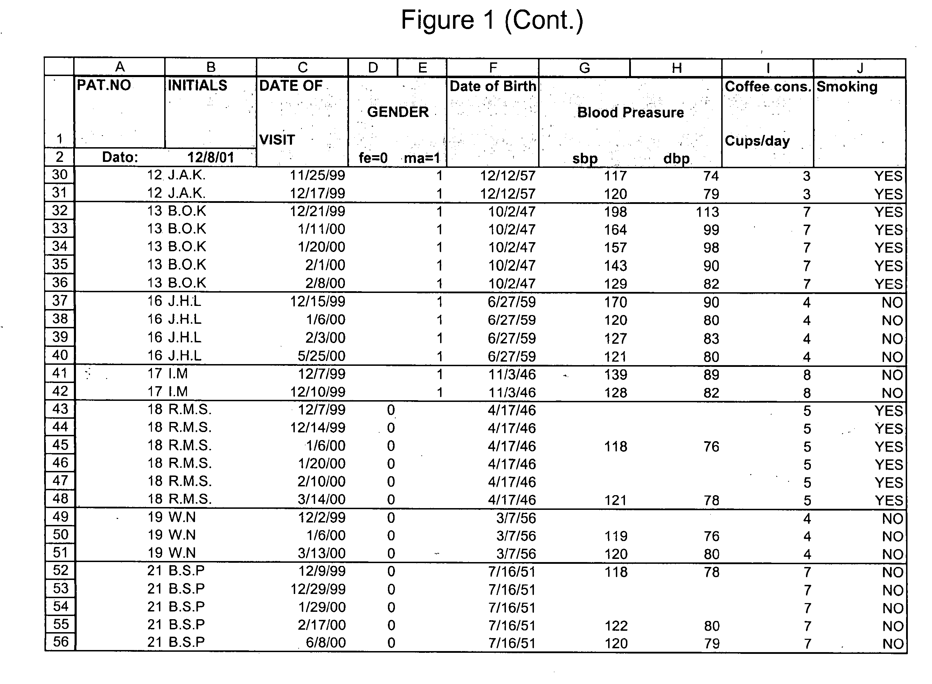

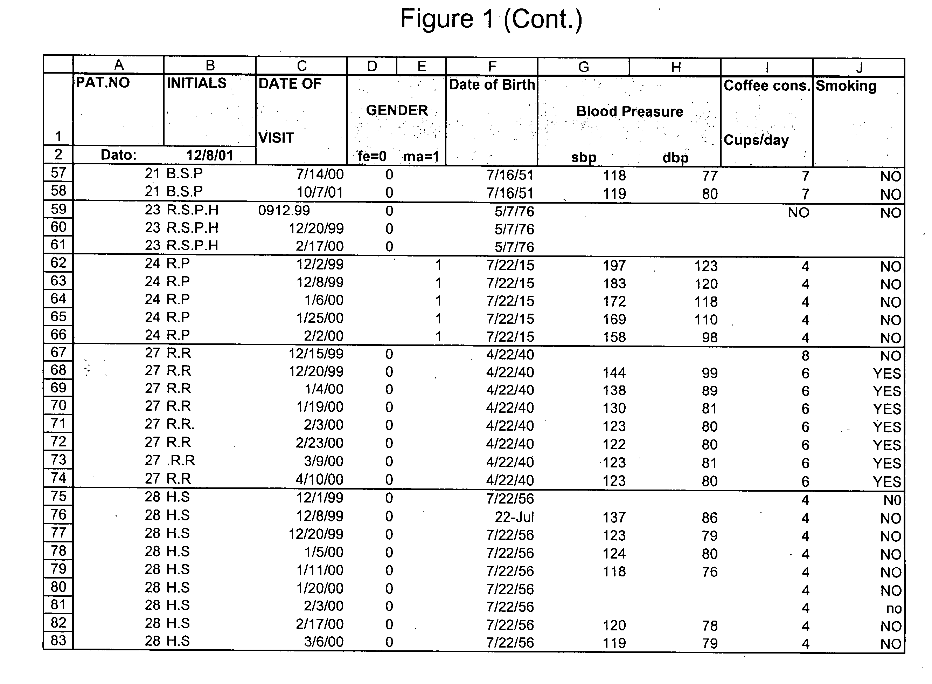

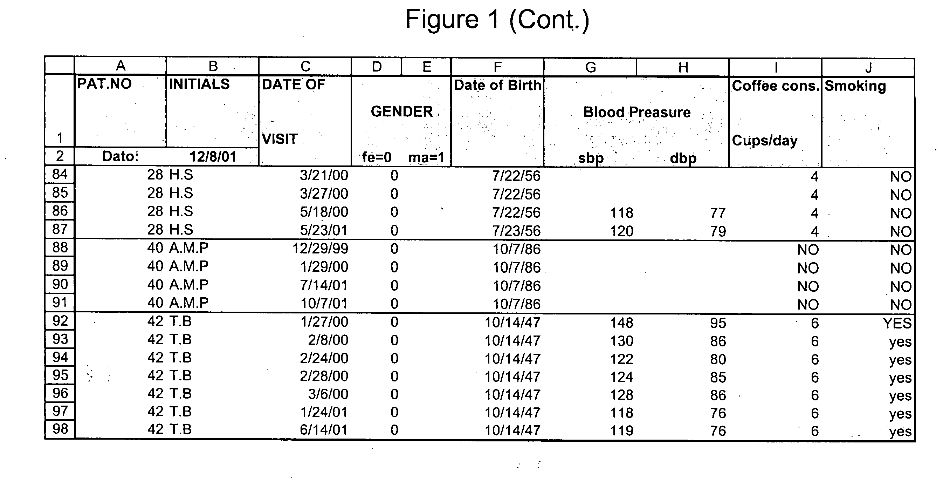

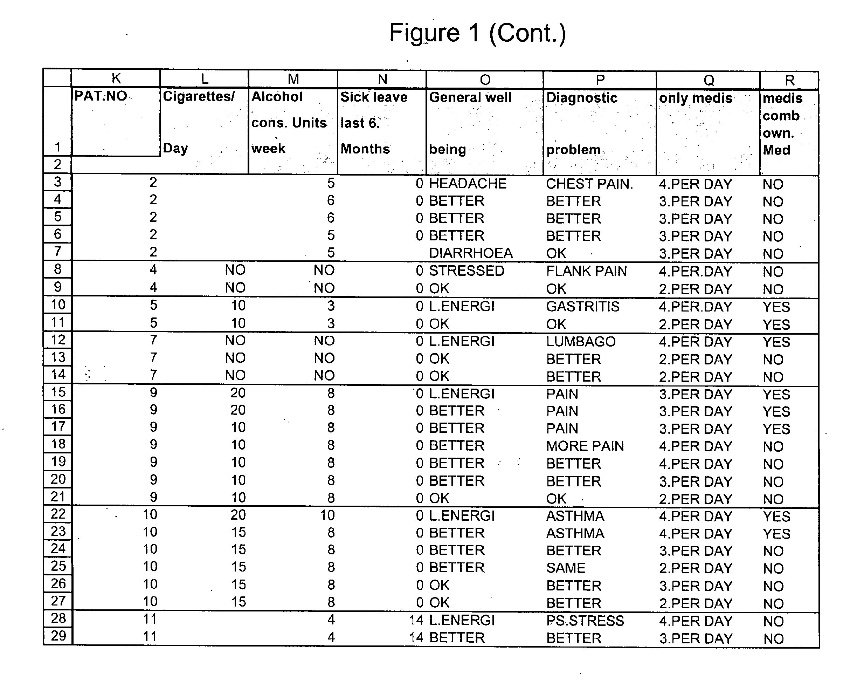

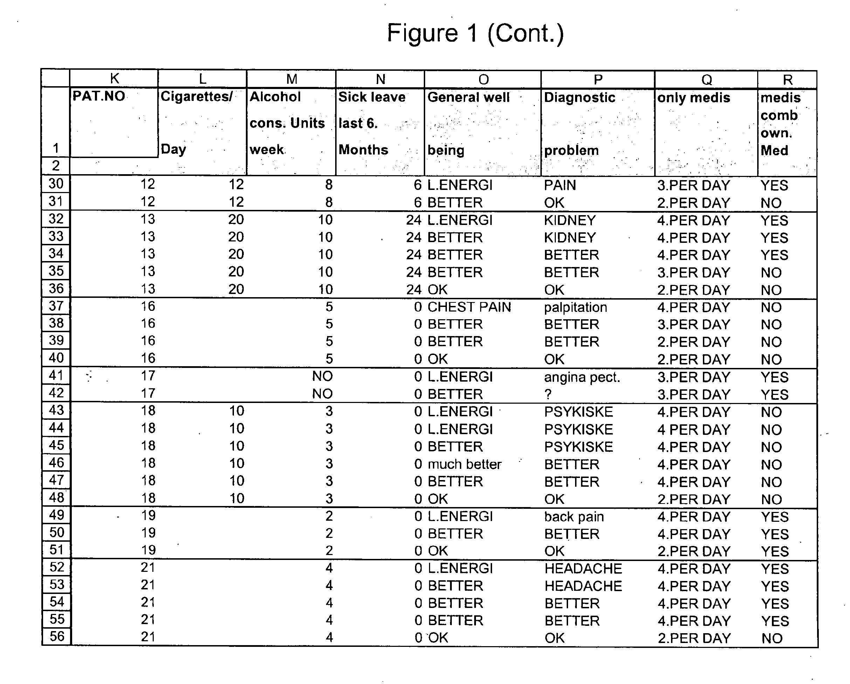

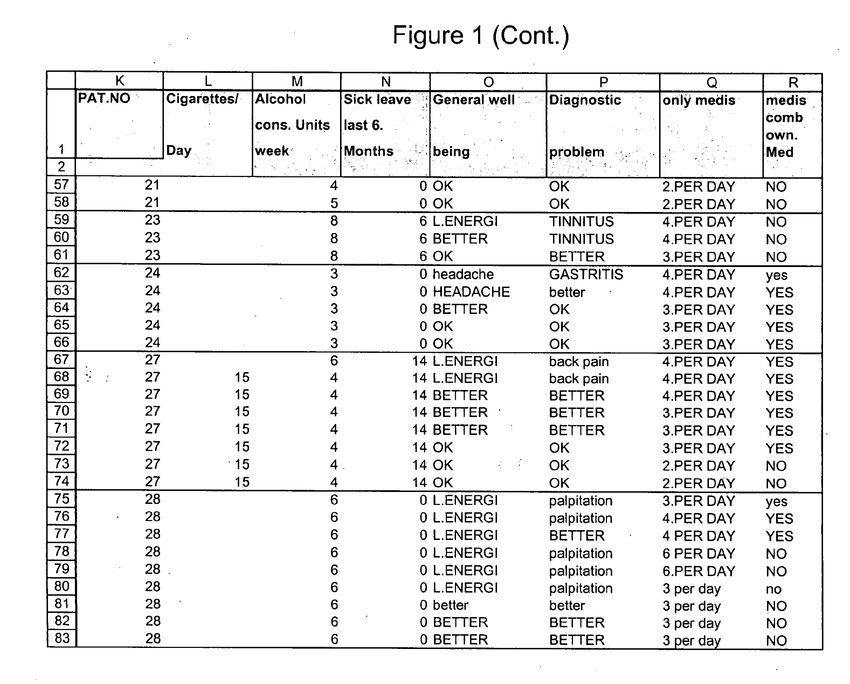

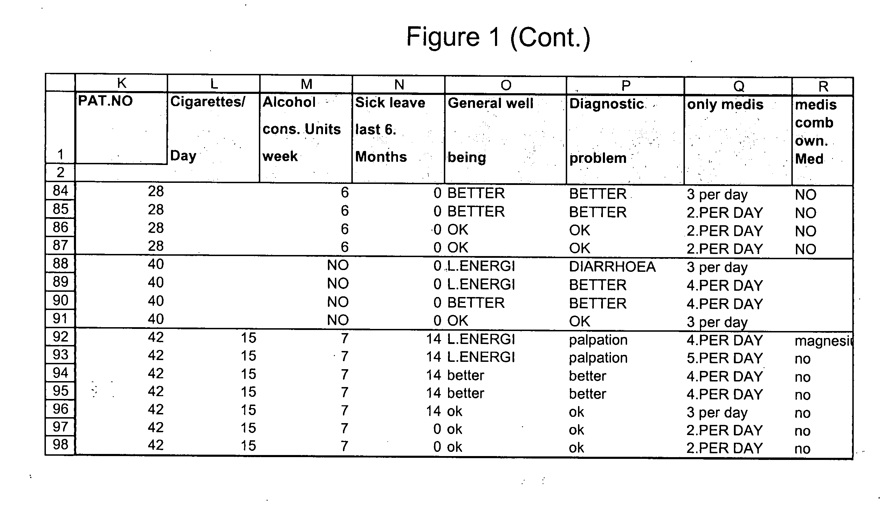

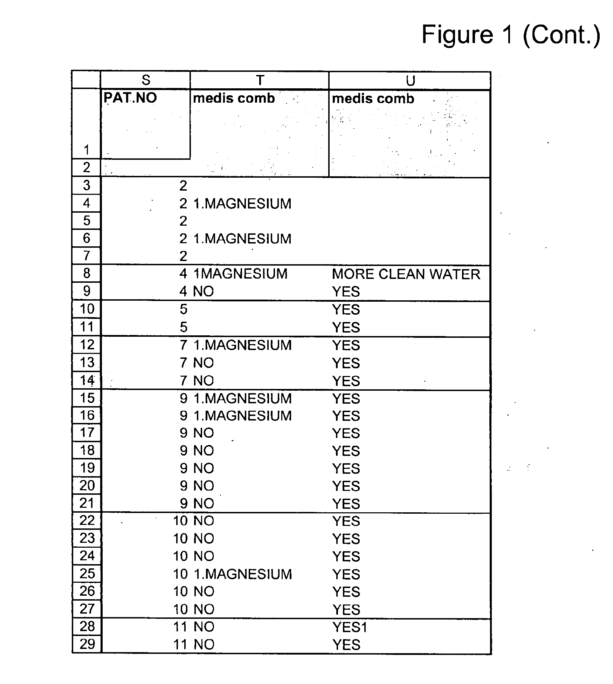

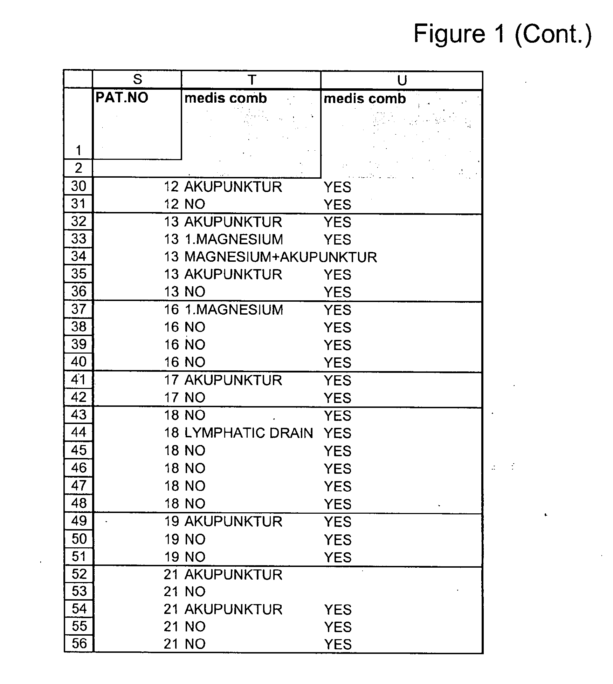

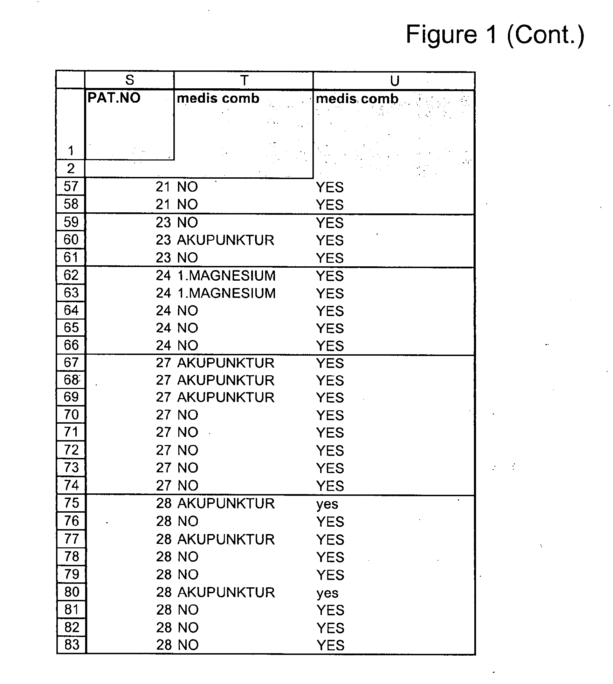

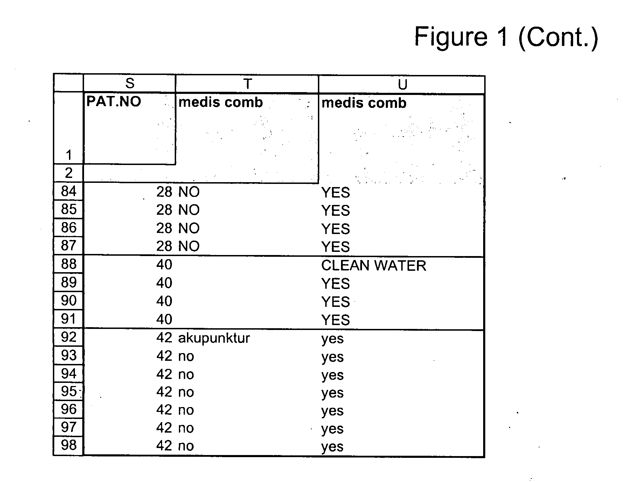

Figure 1 provides a Table of patient data for patients consuming the dietary supplements of the present invention.

### DEFINITIONS

To facilitate an understanding of the invention, a number of terms are defined below.

As used herein, the term "growth medium" refers to a solution used to culture microorganisms. In preferred embodiments, "growth medium" is substantially free of the microorganisms grown in the media. By "substantially free" it is meant that about greater than 90% of the biomass of the microorganisms (*e.g.*, fungi or bacteria) have been removed from the medium, preferably greater than about 95% of the biomass of the organisms have been removed from the medium, more preferably greater than about 99% of the biomass of the organisms has been removed from the medium, and most preferably greater than about 99.9% of the biomass of the organisms has been removed from the medium.

As used herein the term "microorganism" refers to microscopic organisms and taxonomically related macroscopic organisms within the categories of algae, bacteria, fungi (including lichens), protozoa, viruses, and subviral agents.

As used herein, the term "dietary supplement" refers to a product taken orally that contains an ingredient intended to supplement the diet. "Dietary ingredients" in these products include, but are not limited to vitamins, minerals, herbs or other botanicals, amino acids, and substances such as enzymes, organ tissues, glandulars, and metabolites. Dietary supplements can take the form of extracts and concentrates and can be provided as tablets, capsules, softgels, gelcaps, liquids, or powders. Dietary supplements may also be provided in bars, drinks, shakes and other food products. In general, a dietary supplement is not intended to be the sole item of a meal or diet.

As used herein, the term "food product" refers to any food or feed suitable for consumption by humans, non-ruminant animals, or ruminant animals. The "food product" may be a prepared and packaged food (*e.g.*, mayonnaise, salad dressing, bread, or cheese food) or an animal feed (*e.g.*, extruded and pelleted animal feed or coarse mixed feed). In some embodiments, food products further comprise dietary supplements. "Prepared food product" means any pre-packaged food approved for human consumption.

As used herein, the term "foodstuff" refers to any substance fit for human or animal consumption.

As used herein, the term "nutritional supplement" refers to a composition comprising a "dietary supplement" in combination with a source of calories. In some embodiments, nutritional supplements are meal replacements or supplements *(e.g.,* nutrient or energy bars or nutrient beverages or concentrates).

As used herein, the term "oral delivery vehicle" refers to any means of delivering a pharmaceutical or dietary supplement orally, including, but not limited to, capsules, pills, tablets and syrups.

As used herein, the term "cell culture" refers to any *in vitro* culture of cells.

As used herein the term, *"in vitro"* refers to an artificial environment and to processes or reactions that occur within an artificial environment. *In vitro* environments can consist of, but are not limited to, test tubes and cell cultures. The term "*in vivo"* refers to the natural environment (*e.g.*, an animal or a cell) and to processes or reaction that occur within a natural environment.

As used herein, the term "immunoglobulin" refers to proteins that bind a specific antigen. Immunoglobulins include, but are not limited to, polyclonal, monoclonal, chimeric, and humanized antibodies, Fab fragments, F(ab')₂ fragments, and includes immunoglobulins of the following classes: IgG, IgA, IgM, IgD, IbE, and secreted immunoglobulins (sIg). Immunoglobulins generally comprise two identical heavy chains and two light chains.

### DETAILED DESCRIPTION OF THE INVENTION

The present invention relates to dietary supplements. In particular, the present invention provides compositions and methods utilizing dietary supplements derived from fungi and bacteria co-culture growth media.

### I Growth Media

In some embodiments, the present invention provides growth media derived from the growth of microorganisms. The growth media of the present invention comprises an aqueous liquid enriched by components from microorganisms grown in the media (*e.g.*, extracellular components secreted by the microorganisms). The enriched growth media are useful as dietary supplements and as ingredients of food products and foodstuffs. The present invention is not limited to a particular mechanism. Indeed, an understanding of the mechanism is not necessary to practice the present invention. Nonetheless, it is contemplated that the growth media of the present invention is useful in stimulating the immune system.

### A Aqueous Component

The growth media of the present invention comprise an aqueous base with one or more optional ingredients. In some embodiments, the media are "poor" media comprising a solution of glucose in water. The present invention is not limited to glucose. It is contemplated that other mono and disaccharides may be used in the growth media of the present invention. Media comprising additional sugars may be screened for activity using any suitable method including, but not limited to, those described in the illustrative examples below. In other embodiments, the media is enriched to make a "rich" media. For example, in some embodiments, in addition to glucose and water, the media contains tea. In preferred embodiments, the tea is black tea (*e.g.*, Earl Grey tea).

### B Micro Organisms

The growth media of the present invention are media in which one or more microorganisms have been grown. In some embodiments, the organisms are fungi. The present invention is not limited to particular fungi. Any suitable fungi may be utilized, including but not limited to, *Zygosaccharomyces* species. In some preferred embodiments, the organisms are a co-culture of bacteria and fungi. The present invention is not limited to particular bacteria. Any suitable bacteria may be utilized, including but not limited to *Gluconacetobacter europaeus* and *Bacillus pumilus.*

In some preferred embodiments, the fungi and bacteria are in a symbiotic co-culture. The yeasts produce alcohol from sugar during fermentation. The bacteria transform the alcohol to organic acids. The organic acids then act as protective agents for the yeasts against other competing yeast species. The preferred yeast species *Zygosaccharomyces* are characterized by their relatively high tolerance against organic acids. As will be recognized by those skilled in the art, other species of fungi characterized in having a high tolerance to organic acids may be substituted for or used in addition to *Zygosaccharomyces* in the cultures. The present invention is also not limited to a particular bacteria. Any bacteria that generates organic acids from alcohol may be used in the growth media of the present invention.

When grown together in one of the growth media described above, the colonies of bacteria and fungi form fibrous (cellulose) medusa-like discs composed of several membranes in which the bacteria and yeast cells are embedded. Example 1 below describes an exemplary culture method. One skilled in the art recognizes that other suitable culture methods that result in the symbiotic growth of the co-culture may be utilized in the methods and compositions of the present invention. In preferred embodiments, the microorganisms are removed from the media prior to use.

### II Dietary Supplements

In some embodiments, the growth media of the present invention find use as dietary supplements. In preferred embodiments, dietary supplements are prepared by removing the water from the growth media of the present invention to generate a solid (*e.g.*, powder) or oil containing the non-aqueous components of the media. The water may be removed using any suitable method known in the art, including but not limited to, spray-drying with or without an excipient, lyophilization, freeze-drying, vacuum or heat. Preferably, the powder thus obtained is easily dispersible in liquids. In some embodiments, the powder is mixed with a liquid and the liquid consumed orally.

The dietary supplements of the present invention can be provided in a variety of forms. In some embodiments, administration is oral. For oral administration, dietary supplements may be formulated with suitable carriers such as starch, sucrose or lactose in tablets, pills, dragees, capsules, solutions, liquids, slurries, suspensions and emulsions to form a suitable oral delivery vehicle. The tablet or capsule of the present invention may be coated with an enteric coating which dissolves in the digestive tract. A suitable enteric coating which dissolves in the small intestine but not in the stomach is cellulose acetate phthalate. In some embodiments, the dietary supplement is provided as soft gelatin capsules. The dietary supplements may also be provided by any of a number of other routes, including, but not limited to, intravenous, intramuscular, intra-arterial, intramedullary, intrathecal, intraventricular, transdermal, subcutaneous, intraperitoneal, intranasal, enteral, topical, sublingual or rectal means. Further details on techniques for formulation for and administration and administration may be found in the latest edition of *Remington's Pharmaceutical Sciences* (Maack Publishing Co., Easton, PA).

An effective amount of dietary supplements may also be provided as a supplement in various food products, including animal feeds, and drinks. In some preferred embodiments, the dietary supplements of the present invention comprise approximately 3-5 grams of dried, powdered growth media per 1000 grams of food product. For the purposes of this application, food products containing dietary supplements of the present invention means any natural, processed, diet or non-diet food product to which exogenous dietary supplement has been added. Dietary supplements may be directly incorporated into various prepared food products, including, but not limited to beverages (*e.g*., diet drinks, milk, juice), diet bars, supplements, prepared frozen meals, candy, snack products *(e.g.,* chips), prepared meat products, cheese, yogurt and any other foods or beverages.

In some embodiments, the dietary supplements are included in foods that are heated (*e.g.*, breads or other baked goods). It is contemplated that dietary supplements comprising growth media of the present invention are stable to heating (See *e.g.*, Example 6 for methods of assaying temperature stability). Thus, it is contemplated that the growth medium of the present invention or dietary supplements comprising the growth medium may be added to prepared foods prior to heating.

In some embodiments, the food product comprising growth media, or the growth media itself, is heated prior to use. The present invention is not limited to a particular mechanism.

Indeed, an understanding of the mechanism is not necessary to practice the present invention. Nonetheless, it is contemplated that heating may enhance the properties (*e.g.*, the immune stimulating properties) of the growth media of the present invention.

In some embodiments, the dietary supplements comprise one or more additional components including, but not limited to, dry red wine extract, dry shizandra extract, alfalfa, and papain. For example, in some embodiments, supplements comprise dry growth media and dry red wine extract at a ratio of approximately 2.35:1 *(e.g.,* 235 mg dry growth media extract and 100 mg dry red wine extract per tablet). In other embodiments, the dietary supplements comprise dry growth media and dry shizandra extract at a ratio of approximately 4.7:1 (*e.g.*, 235 mg dry growth media and 50 mg shizandra extract per tablet). In still further embodiments, the dietary supplements comprise dry growth media, alfalfa, and papain at a ratio of approximately 7.8:1.67:1 (*e.g.*, 235 mg dry growth media, 50 mg alfalfa, and 30 mg papain per tablet).

### III Immune Stimulation

In some embodiments, the present invention provides compositions (*e.g.*, the dietary supplements described above) for use in stimulating the immune system. Experiments conducted during the development of the present invention (*See e.g.*, Example 3) revealed that growth media of the present invention stimulated T-cell proliferation *in vitro.* T-cell proliferation is involved in the immune response to antigens and vaccines. Accordingly, it is contemplated that the dietary supplements of the present invention will facilitate the immune response against pathogens and the development of immunity in response to vaccination.

Supplements may be administered in any of the forms described above. Dosing is dependent on the particular immune response being modulated, with the course of treatment lasting from several days to several months. Optimal dosing schedules can be calculated from measurements of supplement accumulation in the body of the patient. Optimum dosages may vary depending on the relative potency of the supplements and can generally be estimated based on EC₅₀ found to be effective in *in vitro* and *in vivo* animal models or based on the examples described herein *(e.g.,* Example 3). In general, dosage is from 0.01 µg to 100 g per kg of body weight, and may be given once or more daily, weekly, monthly or yearly.

### IV Animal Feeds

In other embodiments, growth media of the present invention *(e.g.,* dried growth media) is incorporated in animal feeds (*e.g.*, live stock or domestic animal feeds). Suitable formulations of animal feeds are known to those skilled in the art. Many different feed rations may be formulated for animals from many different feed ingredients. Rations are generally formulated to provide-nutrients in accordance with National Research Council standards. The feedstuffs used in the ration are chosen according to market price and availability. Thus, some components of the ration may change over time. In the feeds of the present invention, the ration will always contain growth media, but other components may vary over time based on the price of the component. For discussions on feed ration formulation, actual rations and NRC guidelines, see Church, Livestock Feeds and Feeding, O&B Books, Inc., Corvallis Oreg. (1984) and Feeds and Nutrition Digest, Ensminger, Oldfield and Heineman eds., Ensminger Publishing Corporation, Clovis, Calif. (1990), incorporated herein by reference.

The animal feed rations of the present invention may be characterized according to NRC requirements. NRC requirements may be found in Church, Livestock Feeds and Feeding, O&B Books, Inc., Corvallis Oreg. (1984), or other nutritional standards. Hog and other animal rations are traditionally balanced using the protein and energy requirements, and then adjusted if needed to meet the other requirements. The hog and other feeds of the present invention will contain about 0.05% to 5% lipids plus other feed materials necessary to balance the feed to meet the NRC requirements for the different stages of growth and maintenance.

The relative amounts of protein and energy are adjusted to reflect Nutritional Standards requirements. The amounts of feed components will vary with the stage of animal fed. A growing ration for young animals will have higher protein levels, while a finishing ration for finishing animals for market will have higher energy values which are supplied by carbohydrates. For example, hog prestarter, starter and grower-finisher rations will generally contain about 20-24% protein, 18-20% protein and 13-17% protein respectively. In some feeding situations, care must be taken to provide the appropriate amino acids as well as overall protein content. For example, hogs fed large amounts of corn must have adequate lysine made available in the ration. In most animal diets, energy requirements are met by starches in cereal grains. Energy requirements may also be met by addition of fat to the ration. Addition of fat to hog rations has been proven to increase growth rate slightly. Feed intake is reduced and feed efficiency is improved when fat is added to the ration. Since feed intake is reduced when fat is added, it important to increase the level of protein so that the daily intake of protein is maintained. In general, the protein level should be increased by 0.2% for every 1% addition of dietary fat.

### EXPERIMENTAL

The following examples serve to illustrate certain preferred embodiments and aspects of the present invention and are not to be construed as limiting the scope thereof.

In the experimental disclosure which follows, the following abbreviations apply: M (molar); mM (millimolar); µM (micromolar); nM (nanomolar); mol (moles); mmol (millimoles); µmol (micromoles); nmol (nanomoles); gm (grams); mg (milligrams); µg (micrograms); pg (picograms); L (liters); ml (milliliters); µl (microliters); cm (centimeters); mm (millimeters); µm (micrometers); nm (nanometers); ºC (degrees Centigrade).

### Example 1

### Generation of Growth Media

The species composition of the microbes in the growth media is comprised of the following microbes.

### Bacteria:

Gluconacetobacter europaeus
Bacillus pumilus

### Fungi:

### Zygosaccharomyces spp. (two different species)

Microorganisms were grown in two different media defined as "rich" and "poor".

### "Poor" medium

The medium is prepared by boiling 3800 ml water and adding 550 g of glucose. The glucose is then solubilized in the water.

### "Rich" medium

This medium is prepared exactly the same way as "poor" medium. In addition, 80 g of Earl Grey black tea is added.

Samples of rich and poor media in which no organisms have been grown were used as negative controls when screening for antimicrobial and immuno stimulating effects (Examples 2 and 3).

### Growth conditions:

Growth medium (3800 ml) was poured into a 150 mm x 150 mm x 150 mm steel container. The "mother" mycelium (a matrix measuring 150 mm x 150 mm x10 mm) of cellulose fibers produced by the bacteria, which embeds the fungi and bacteria) was carefully layered on top of the medium. The culture was grown in an incubator at 25°C with a normal atmosphere of 80% relative humidity. After 3 days a new membrane ("child") was visible on top of the "mother" mycelium. The new mycelium was separated from the "mother" by a gas layer. After 2-3 more days the gas penetrated the bottom layer (mother). After ten days of growth the new mycelium ("child") was separated from the "mother". The two layers ("mother" and "child") of cellulose fibers were removed from the growth medium and stored in containers at 5°C to rest for 14 days before the growth cycle is repeated with each of the mycelia ("mother" and "child"). The growth medium in which the microorganisms had been grown was used in the screening tests described below. Both "rich" and "poor" growth media were used with corresponding negative controls (above) when screening for antimicrobial and immuno stimulating effects (See Examples 2-3 below).

### Example 2

### Anti-Microbial Effects

Microorganisms were collected from Culture Collection, University of Göteborg, Sweden (CCUG). For microorganisms that can be cultivated on agar plates the organism was grown on an appropriate medium. The organism was then suspended in physiological saline (or peptone water for dermatophytes) using the following method: *Pneumococcus, Streptococcus, Enterococcu,* and *Hemofilus* cultures were diluted 20 colonies to 1 ml NaCl. One drop of the resulting solution was then diluted into 5 ml of NaCl. *Staphylococcus* cultures were diluted 10 colonies to 1 ml of NaCl. One drop of the resulting solution was then diluted into 5 ml of NaCl. Cultures of Gram negative rods were diluted 2-3 colonies to 1 ml NaCl. One drop of the resulting solution was then diluted into 5 ml of NaCl. Cultures of yeasts were diluted 1-2 colonies to 1 ml NaCl. The resulting solution was then diluted 1 drop to 5 ml NaCl.

A fresh agar plate was flooded with the suspension from the 5 ml tubes. The excess was pipetted off and the agar plate was allowed to dry for approximately 30 min. Wells were then made in the agar plates. Drops with different concentrations of growth media were then added to the wells in the agar plates (20 µl of each concentration). In addition, glycerol only samples were used as reference samples. The plates were incubated under appropriate conditions and read after a predetermined time. The zone of clearing around each well was observed and scanned. At least two replicate assays were carried out for each organism.

Table 1 shows the result of the screening assays. Two different growth media were tested for their ability to inhibit the growth of bacteria and fungi. One of the media was defined as rich and the other as poor. The negative control was growth media that did not have microbial culture grown in it. The effect was scored using a scale from 0 (none) to +++ (large inhibition zone).

**Table 1**

| **Microbe** | **Rich Media +microbes** | **Rich Media +microbes** | **Rich medium** | **Poor medium** |
|---|---|---|---|---|
| Staphylococcus epidermis | 0 | 0 | 0 | 0 |
| *Staphylococcus aureus* | 0 | 0 | 0 | 0 |
| *Methiclllin resist. S. aureus* | 0 | 0 | 0 | 0 |
| *Staphylococcus saprophyticus* | 0 | 0 | 0 | 0 |
| *Escherichia coli* | 0 | 0 | 0 | 0 |
| *Enterococcus faecalis* | 0 | 0 | 0 | 0 |
| *Moraxella catharralis* | 0 | 0 | 0 | 0 |
| *Neisseria meningitides Gr.B* | 0 | 0 | 0 | 0 |
| *Streptococcus dysgalactia Gr.G* | 0 | 0 | 0 | 0 |
| *Streptococcus pneumonia* | 0(+) | 0 | 0 | 0 |
| *Hemophilus influenzae* | 0 | 0 | 0 | 0 |
| *Arcanobacterium haemolyticum* | ++ | ++ | 0 | 0 |
| *Legionella pneumophilia* | + | 0 | 0 | 0 |
| *Pseudomonas aeruginosa* | (+) | 0 | 0 | 0 |
| *Propionibacterium acnes* | 0 | 0 | 0 | 0 |
| Acinetobacter *calcoaceticus* | | | | |
| *Candida albicans* | 0 | 0 | 0 | 0 |
| *Saccharomyces cerevisiae* | 0 | 0 | 0 | 0 |
| *Aspergillus fumigatus* | 0 | 0 | 0 | 0 |

### Example 3

### Screening Assays for Immune Stimulating Responses

This Example describes the screening of the growth media of the present invention for their ability to stimulate a series of different immune responses.

### A Analysis of Macrophage Stimulation

This assay involves measuring the production of interleukin-1. Macrophages are cells involved in immune responses and are generally stimulated by antigens. The present invention is not limited to a particular mechanism. Indeed, an understanding of the mechanism is not necessary to understand the present invention. However, it is contemplated that a wide range of different polysaccharides may act as mitogen stimulating agents. Stimulation of macrophages induces production of Interleukin-1 (IL-1), an immune regulating factor. The presence of IL-1 can be measured by ELISA (Enzyme-Linked-Immuno-Sorbent-Assay). Increased production of IL-1 (relative to neg. control) is indicative of stimulation of macrophages my growth media.

For all assays leucocytes were prepared from human blood samples using Lymphoprep and density gradient centrifugation using the standard procedure given by Nycomed (Linz, Austria). The lymphocytes were grown in a standard growth medium (Fetal calf serum). Plastic adherent cells were used for the assay. An IL-1 microtiter plate was utilized that was a 96 wells polystyrene microtiter plate covered with monoclonal antibodies with specificity against human IL-1. Detection of IL-1 was achieved by incubating polyclonal antibodies with specificity against human IL-1 conjugated with Horse-radish-peroxidase. The chromogen substrate used in the enzyme reactions was tetramethylbenzidine, which gives a colored product that can be read optically as absorbance at 280 nm.

The wells were incubated with growth medium from the macrophage cultures (containing IL-2) and the growth media of the present invention in different dilutions (1:10, 1:50, 1:100, 1:500 and 1:1000). Negative controls are defined as "poor" (sugar in water) and "rich" (sugar, tea and water) media in which nothing had been grown. Positive controls were Lipopolysaccharide (LPS) from *E.coli* stimulated macrophages. Lipopolysaccharides from gram-negative bacteria are known to stimulate monocytes, macrophages and B-cells.

Weak stimulation was observed, but no significant elevated levels of IL-1 from any of the tested media. Significant effects were defined as values greater than mean values of unstimulated cells plus twice the value of standard deviation (SD).

### B Analysis of B-cell Stimulation via Proliferation Assay

Proliferation of lymphocytes takes place *in vivo* as a reaction to antigens, infections and vaccines. Cell division of lymphocytes can be detected *in vitro* using different methods involving antigens or mitogens. Standard procedures were used in these assays. As a positive control, LPS was used to stimulate B-cells. The DNA-base analogue, 5-bromo-2-deoxyuridine (BdU) was incorporated into *de novo* synthesized DNA, and used as a measure for cell division (proliferation). Incorporated BdU was measured using an ELISA with monoclonal antibodies with specificity against BdU conjugated to Horseradish Peroxidase. Weak proliferation and incorporation of BdU was observed.

### C Analysis of T-cell stimulation

As a measure for cell division and *de novo* synthesis of DNA, incorporation of ³H-thymidine was monitored. The cell cultures were stimulated by growth media of the present invention in the presence of ³H-thymidine. After 2-5 days of incubation the cells were washed and the sample was monitored for incorporated radioactivity using scintillation counting. Briefly, 2 million cells were grown in traditional medium. Growth media of the present invention and ³H-thymidine were then added. Cells were then washed in buffer to remove excess radioactivity, followed by scintillation monitoring.

Results are shown in Table 2. Significant proliferation of T-lymphocytes was observed using three different growth media (sterile water and sugar; sterile water, sugar and black tea; tap water, sugar and black tea) diluted 1:10. The negative control was sterile water and sugar (550 gr. glucose to 3800 ml water). The positive control was Interleukin 2.

**Table 2**

| Sample no 1 | Cpm ³H-thym | Sample no 2 | Cpm ³H-thym | Sample no 3 | cpm ³H-thym |
|---|---|---|---|---|---|
| Neg. contr. | 1708 | Neg. contr. | 1708 | Neg. contr. | 1708 |
| Int-leu-2 | 31 820 | Int-leu-2 | 31 820 | Int-leu-2 | 31 820 |
| Med1 1:10 | 2463 | Med2 1:10 | 2494 | Med3 1:10 | 3437 |
| Med1 1:50 | 1720 | Med2 1:50 | 924 | Med3 1:50 | 961 |
| Med1 1:100 | 774 | Med2 1:100 | 137 | Med3 1:100 | 78 |
| Med1 1:500 | 291 | Med2 1:500 | 117 | Med3 1:500 | 66 |
| Med1 1:1000 | 71 | Med2 1:1000 | 114 | Med3 1:1000 | 45 |

In order to investigate the molecule(s) responsible for the stimulating effect shown in Table 2, 1 ml of the different growth media extracts (Med1, Med2 and Med3) were centrifuged at 12,000 rpm in order to precipitate micro organisms. The supernatants were sterile filtered and applied to a gel filtration column (HPLC). The absorbance at 206 and 280 nm was monitored in the eluted fractions. The absorbance was approximately zero (0) for all the fractions. Three different fractions (low-, medium and high molecular weight) were tested for leukocyte stimulating effects after being concentrated 5x. All the fractions were negative, *i.e.* gave no significant increase in proliferation rates.

SDS-PAGE analysis of the three different media were also negative, i.e. no visible bands occurred after staining for proteins. These data verify the absorbance monitored in the fractions resulting from the above HPLC-gel filtration studies. The pellet from Med3 (micro organisms in sterile water) showed the presence of polysaccharides/glyco-proteins (distinct bands on the gel). Additional experiments were carried out in which monocultures isolated from the complex micro organism medium liquid mixture were grown in standard SABOROUD medium (containing antibiotic agents). When these growth media were tested for leukocyte stimulating effects the response was substantial. However, which class of leukocytes (T-cells, B-cells, macrophages etc.) was not determined.

In conclusion, both SDS-PAGE and absorbance at 280nm and 206nm after HPLC fractionation failed to detect any traces of possible active ingredients. The above-described procedure has a relatively high sensitivity (0,1 ng/ml) with respect to detection of proteins and carbohydrates. The present invention is not limited to a particular mechanism. Indeed, an understanding of the mechanism is not necessary to practice the present invention. Nonetheless, it is contemplated that the positive stimulating effects of T-lymphocytes indicates the presence of a very potent T-lymphocyte stimulating agent.

### Example 4

### Safety in Humans

A dietary supplement comprising growth media of the present invention was administered to over 3000 patients. The individual treatments reached from several weeks to 1.5 years. The product was administered orally as capsules that contained the lyophilized growth medium.

The content of one capsule corresponds to 1,66 ml lyophilized growth medium and 235 mg dry weight (ca. 100 mg silica included). The content of bacteria in the final product is below FDA's defined limits for food and dietary supplements (less than 10 bacteria per mg).

Figure 1 shows a table of data on 20 randomly picked patients that were subjected to treatment with the growth media of the present invention. The columns give different relevant personal data:1^{st}: Initials of patient; 2^{nd}: Dates of visits; 3^{rd}: Gender; 4^{th}: Date of birth; 5^{th}: Blood pressure (systolic and diastolic); 6^{th}; Coffee consumption, cups per day; 7^{th}: Smoker/Non smoker; 8^{th}: Cigarette consumption, cigs. per day; 9^{th}: Alcohol consumption, units pr. week; 10^{th}: Sick leave from job last six months, days; 11^{th}: General well being; 12^{th}; Diagnosis; 13^{th}: growth media administered, number of capsules; 14^{th}; Media combined with the patients own medicine *i.e.* Western traditional school medicine; 15^{th}: Media combined with different sort of treatment *i.e.* magnesium, acupuncture, lymphatic drainage etc.; 16^{th}: Media combined with clean water.

The data indicate that most of the patients were cured for their clinical conditions at the end of treatment (12^{th} column). In addition, none of the patients gained any new problems, new symptoms or unintentional side effects after being medicated with the growth media of the present invention for over a year (11^{th} column). Further, during the trial period no negative effects as a consequence from using the media of the present invention were detected. In conclusion, the growth media of the present invention was demonstrated to be safe and does not represent any health risk when administered orally (in specified doses) as a dietary supplement for humans.

### Example 5

### Assay for the Active Components of Growth Medium

In order to find out the nature of the bioactive substance in the growth medium of the present invention, experiments are designed to determine whether it has characteristics of a protein or a carbohydrate. A standard proliferation assay of T-lymphocytes measuring *de novo* synthesis of DNA by incorporation of H³-Thymine (See Example 3) is used.

### Positive controls:

### Interleukin I

Sterile filtered growth medium of the present invention.

### Negative control:

Sterile filtered growth medium of the present invention without fungus.

### Experimental groups:

1) Sterile filtered growth medium is treated with a general protease in order to degrade all proteins in the medium. The treated growth medium is then tested for activity. Lack of activity indicates a protein nature of the growth medium. Unchanged activity indicates a non-protein nature of the growth medium.
2) Sterile filtered growth medium is treated with a Con-A column. The flow-through is tested for activity. Lack of activity indicates carbohydrate nature of the growth medium. Unchanged activity indicates a non-carbohydrate nature of the growth medium.
3) If biological activity is retained on the Con-A column, the bound material is washed through using high ionic strength buffer. The effluent is then concentrated and tested for activity. The presence of biological activity is indicative of a carbohydrate nature of the growth medium.
4) All tested media is analyzed on PAGE and stained for proteins and carbohydrates respectively.

It is contemplated that the results of the present experiment will indicate that the active ingredient in the growth medium of the present invention is a micro heterogeneous carbohydrate residue with a molecular weight of approx. 60 kD. It is further contemplated that the residue is a soluble component normally integrated in the chitin fungus cell wall.

### Example 6

### Assay for Biological Activity of Growth Medium Capsules

Growth medium is subjected to a drying process in which the soluble components are converted into a powder and encapsulated in a gelatine container. A dose response *in vitro* test is done as follows. The test system used is a standard proliferation assay of T-lymphocytes measuring *de novo* synthesis of DNA by incorporation of H³-Thymine (See Example 3). Positive and negative controls described in Example 5 are used.

### Experimental groups:

The content of capsules is dissolved in PBS (Phosphate Buffered Saline) to achieve the following concentrations:
1) 1000 x the concentration of the initial growth medium.
2) 100 x the concentration of the initial growth medium.
3) 10 x the concentration of the initial growth medium.

It is contemplated that the dose response initially will follow a normal linear curve of 1^{st} degree with respect to concentration. It is further contemplated that the response at higher concentrations will deviate from this curve and reach saturation at a certain level. The dose response is expected to follow a hyperbolic saturation curve.

### Example 7

### Heat Stability/Tolerance

In order to map the heat stability of the active components in the growth medium of the present invention, the following test regime is utilized. The test system used is the standard proliferation assay of T-lymphocytes measuring *de novo* synthesis of DNA by incorporation of H³-Thymine described in Example 3.

### Positive controls:

### Interleukin I

Sterile filtered growth medium.

### Negative control:

Heat denatured interleukin I

### Experimental groups:

Dried growth medium (raw material used in growth medium capsules) is dissolved in PBS to a concentration 10x the initial concentration of the medium and tested according to the following regime:

### In liquid (temperatures under 100°C):

1) 60 min at 70°C
2) 5 min at 99°C
3) 10 min at 99°C
4) 20 min at 99°C
5) 40 min at 99°C
6) 60 min at 99°C

After heat treatment, the individual samples are tested for biological activity (ability to stimulate proliferation of T-cells).

### Dry powder (temperatures above 100°C):

Dried growth medium (raw material used in growth medium capsules) is tested according to the following regime:
1) 60 min at 120°C
2) 60 min at 150°C
3) 60 min at 200°C

After heat treatment the powder is dissolved in PBS to a concentration 10 x the initial concentration of the medium and tested for biological activity (ability to stimulate proliferation of T-cells).

It is contemplated that the activity will follow slowly an inverse hyperbolic curve with respect to treatment time. However, the activity is not expected to be reduced significantly or totally lost at temperatures below 120°C.

### Example 8

### Bread Comprising Growth Media

Bread comprising growth media of the present invention was prepared using the following recipe:
4 l water
4.5 kg wheat flour
250 g graham flour
250 g bran/pollard
120 g margarine
350 g yeast
100 g salt
30 g dried and powdered growth media

16-17 loaves of bread (approximately 500 g each) were prepared and baked at 115°C for 29-30 minutes.

All publications and patents mentioned in the above specification are herein incorporated by reference. Various modifications and variations of the described method and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology, microbiology, nutrition, or related fields are intended to be within the scope of the following claims.

## Claims

1. A dietary supplement comprising growth medium from at least one microorganism.

2. The dietary supplement of Claim 1, wherein said microorganism is a fungi.

3. The dietary supplement of Claim 2, wherein said fungi is present as a co-culture.

4. The dietary supplement of Claim 3, wherein said co-culture is a symbiotic co-culture.

5. The dietary supplement of Claim 2, wherein said fungi is *Zygosaccharomyces.*

6. The dietary supplement of Claim 3, wherein said co-culture comprises at least one bacteria.

7. The dietary supplement of Claim 6, wherein said bacteria is selected from the group consisting of *Gluconacetobacter europaeus* and *Bacillus pumilus.*

8. The dietary supplement of Claim 1, wherein said growth medium is dry growth medium.

9. The dietary supplement of Claim 8, wherein said supplement further comprises one or more additional components selected from the group consisting of dry red wine extract, dry shizanda extract, alfalfa, and papain.

10. The dietary supplement of Claim 9, wherein said supplement comprises dry growth medium and dry red wine at a ratio of approximately 2.35:1.

11. The dietary supplement of Claim 9, wherein said supplement comprises dry growth medium and dry shizandra extract at a ratio of approximately 4.7:1.

12. The dietary supplement of Claim 9, wherein said supplement comprises dry growth medium, alfalfa, and papain at a ratio of approximately 7.8:1.67:1.

13. A foodstuff comprising the dietary supplement of any of the Claims 1 to 7.

14. A food comprising the dietary supplement of any of the Claims 1 to 7.

15. The food of Claim 14, wherein said food is a beverage.

16. The food of Claim 14, wherein said food is heated.

17. The food of Claim 16, wherein said growth medium is added to said food before said food is heated.

18. The food of Claim 17, wherein said food is a bread.

19. The food of Claim 14, wherein said food comprises 3-5 g of dry growth media per 1000 g of food.

20. An animal feed comprising the dietary supplement of any of the Claims 1 to 7.

21. The animal feed of Claim 20, wherein said feed is heated prior to use.

22. The animal feed of Claim 21, wherein said feed is a solid.

23. The animal feed of Claim 22, wherein said feed is a liquid.

24. The use of a dietary supplement of any of the Claims 1 to 7 for the manufacture of a medicament for stimulating immune response in a cell.

25. The use of Claim 24, wherein said immune response comprises a T-cell immune response.

26. The use of Claim 25, wherein said cell is a T-lymphocyte.
